# FASCICULE DE BREVET EUROPEEN

(11) **EP 1 622 863 B1**
(45) Date de publication et mention de la délivrance du brevet: **13.08.2008**
(21) Numéro de dépôt: 04742669.7
(22) Date de dépôt: 07.05.2004
(51) Int. Cl.: C07C 253/34

(54) **PROCEDE D HYDROCYANATION DE COMPOSES INSATURES**
VERFAHREN ZUR HYDROCYANIERUNG VON UNGESÄTTIGTEN VERBINDUNGEN
METHOD FOR THE HYDROCYANATION OF UNSATURATED COMPOUNDS

(30) Priorité: 12.05.2003 FR 0305673
(43) Date de publication de la demande: 08.02.2006
(73) Titulaire: RHODIA POLYAMIDE INTERMEDIATES, 69192 Saint-Fons (FR)
(72) Inventeur: ROSIER, Cécile, F-69510 Soucieu en Jarrest (FR); LECONTE, Philippe, F-69330 Meyzieu (FR); BOURGEOIS, Damien, 69003 Lyon (FR)
(74) Mandataire: Esson, Jean-Pierre
(86) Numéro de dépôt international: PCT/FR2004/001110
(87) Numéro de publication internationale: WO 2004/101498

(56) Documents cités:
- WO-A-03/011457
- US-A- 3 773 809
- US-A- 4 539 302

## Description

La présente invention concerne un procédé d'hydrocyanation de composés insaturés en composés mononitriles insaturés ou en dinitriles.

Elle se rapporte plus particulièrement à un procédé de fabrication de dinitriles par double hydrocyanation de dioléfines telles que le butadiène comprenant une récupération et séparation du système catalytique.

La réaction d'hydrocyanation est industriellement utilisée pour la synthèse de composés comprenant des fonctions nitriles à partir de composés comprenant des insaturations. Ainsi, l'adiponitrile, qui est un intermédiaire chimique important notamment dans la fabrication de l'hexaméthylène diamine, monomère pour un certain nombre de polymères comme le polyamide, est fabriqué par hydrocyanation en deux étapes du butadiène ou d'une coupe d'hydrocarbures appelée coupe C4 comprenant du butadiène. Dans ce procédé de fabrication, les deux réactions sont mises en oeuvre avec des systèmes catalytiques comprenant essentiellement les mêmes espèces, à savoir un complexe organométallique de coordination et au moins un ligand organophosphoré de type organophosphite monodentate tel que le tritolylphosphite.

De nombreux brevets décrivent ce procédé de fabrication de l'adiponitrile, ainsi que les procédés de fabrication des catalyseurs.
Par ailleurs, pour l'économie du procédé il est important de pouvoir récupérer le système catalytique et le recycler dans les étapes d'hydrocyanation.
Ainsi, le brevet US 4539302 décrit un procédé de récupération du catalyseur à partir du milieu réactionnel obtenu dans la deuxième étape du procédé de préparation de l'adiponitrile, à savoir l'hydrocyanation de nitriles insaturés en dinitriles.

Ce procédé de récupération par décantation permet de limiter les pertes en élément métallique et facilite le contrôle du rapport ligand organophosphoré/élément métallique pour l'hydrocyanation des nitriles insaturés. Ainsi, on peut, également, récupérer un système catalytique avec un rapport ligand/élément métallique élevé permettant un recyclage et une réutilisation du système catalytique dans les étapes de fabrication de catalyseur et/ou dans les étapes d'hydrocyanation du butadiène ou l'isomérisation des pentènenitriles ramifiés.

De nombreux autres ligands organophosphorés ont été proposés pour la catalyse de ces réactions d'hydrocyanation.

Ainsi, les ligands bidentates de type organophosphite, organophosphinite, organophosphonite et organophosphine ont été décrits dans de nombreux brevets tels que, par exemple, les brevets WO9906355, WO9906356, WO9906357, WO9906358, WO9952632, WO9965506, W09962855, US5693843, WO961182, WO9622968, US5981772, WO0136429, W09964155, WO0213964.

Enfin, il a également été proposé par le brevet WO 03/11457 d'utiliser un mélange de ligands mono et bidentate pour la catalyse des réactions d'hydrocyanation.

Dans le cas de mélanges de ligands, il est également important de pouvoir récupérer le catalyseur sans perdre de ligands ni d'élément métallique. Ainsi, le brevet US 377 3809 décrit l'extraction avec de la paraffine ou de la cycloparaffine d'un complexe organophosphoré du milieu réactionnel. Un des buts de ta présente invention est de proposer un procédé de fabrication de dinitriles par hydrocyanation de composés insaturés utilisant un système catalytique comprenant un mélange de ligands monodentates organophosphites et de ligands bidentates organophosphorés permettant une séparation et récupération du catalyseur et des différents produits avec une perte minimale en composés formant le système catalytique.

A cet effet, l'invention propose un procédé de fabrication de dinitriles par hydrocyanation de composés insaturés comprenant au moins une étape d'hydrocyanation en présence d'un système catalytique comprenant un complexe organométallique formé par un ou plusieurs ligands organophosphorés de type organophosphite monodentate, et un ou plusieurs ligands organophosphorés bidentates et le nickel, le cobalt, le fer, le ruthénium, le rhodium, le palladium, l'osmium, l'iridium, le platine, le cuivre, l'argent, l'or, le zinc, le cadmium, le cérium, et comprenant éventuellement un promoteur de type acide de Lewis, et au moins une étape de séparation par distillation d'un réactif utilisé dans le procédé ou d'un composé formé par la réaction à partir d'un milieu contenant ledit système catalytique, caractérisé en ce que le milieu soumis à l'étape de séparation par distillation comprend un rapport du nombre de moles de ligands organophosphorés monodentates et bidentates exprimé en atome de phosphore par rapport au nombre d'atome d'élément métallique inférieur ou égal à 15 et/ou une concentration pondérale en élément métallique formant le complexe organométallique inférieure ou égale à 1,3 % et en ce que la température de pied de distillation de l'étape de distillation est inférieure ou égale à 180°C.

Par température de pied de distillation, on entend la température du milieu contenu dans le bouilleur de l'installation de distillation, ainsi que la température des parois du bouilleur.

Les conditions de distillation et notamment la température maximale des parois du bouilleur permettent de limiter et même de supprimer la précipitation de l'élément métallique formant le complexe organométallique. En effet, une température plus élevée peut provoquer une décomplexation du catalyseur entraînant la précipitation de l'élément métallique, au cours de l'étape de séparation ou distillation.

Le nickel est l'élément catalytique préféré. Pour plus de clarté, l'élément métallique sera désigné par le terme nickel dans la suite du présent texte, sans que cela ait une signification limitative.

Comme ligands monodentates organophosphites convenables pour l'invention, on peut citer, à titre d'exemple, le triphénylphosphite, le tritolylphosphite (TTP), le tricyménylphosphite.

Comme ligands bidentates convenables pour l'invention, on peut citer les composés organophosphites organophosphonites, organophosphinites, organophosphines et notamment ceux de structures suivantes dans lesquelles Ph signifie phényle :

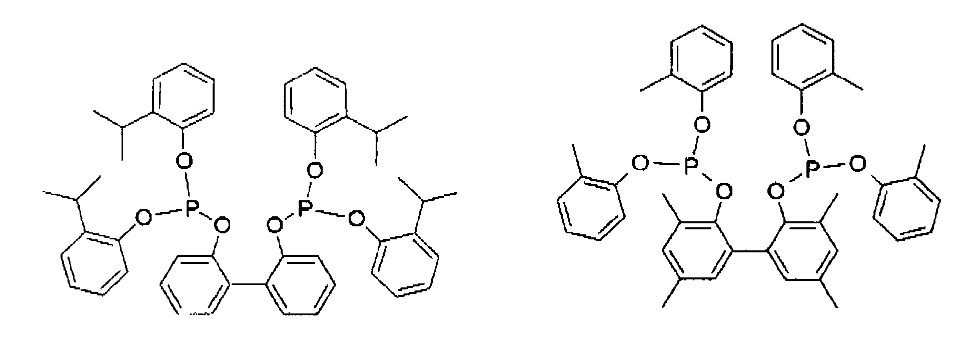

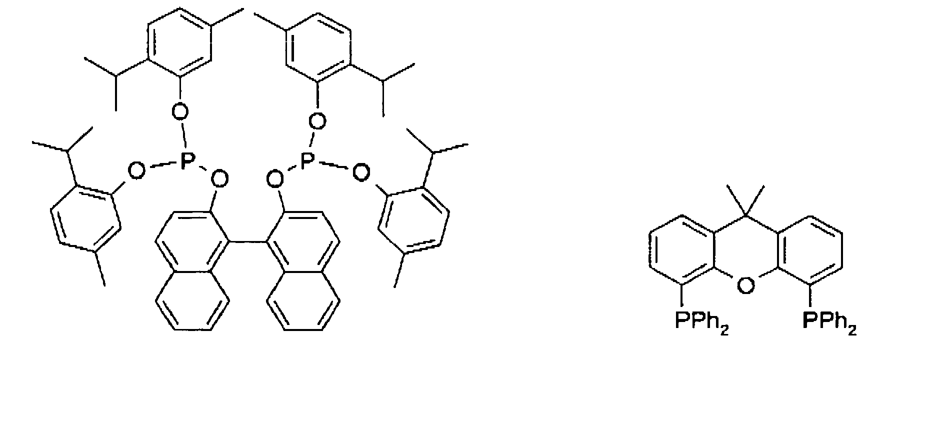

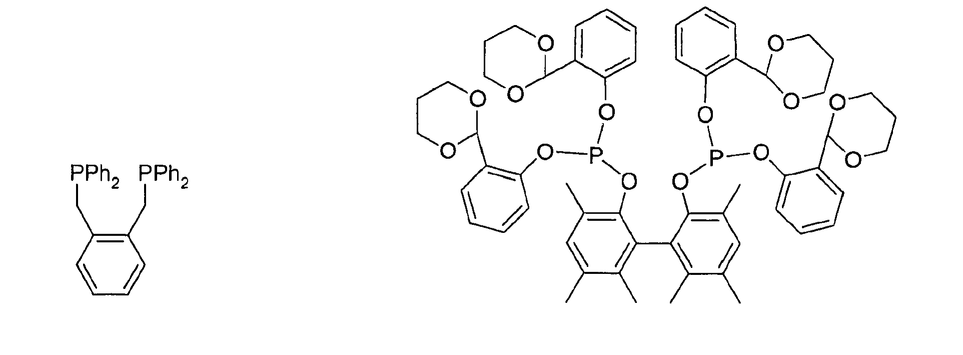

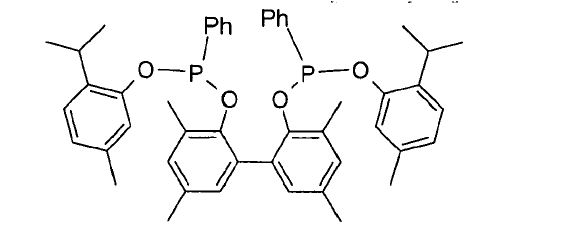

Selon une caractéristique préférentielle de l'invention, le système catalytique présent dans le milieu réactionnel comprend généralement un nombre de moles de ligands bidentates exprimé en nombre d'atome de phosphore par rapport à un atome d'élément métallique compris entre 1 et 4 (bornes incluses), tandis que celui de ligands monodentates exprimé en nombre d'atome de phosphore est compris entre 4 et 12 (bornes incluses).

Ces rapports peuvent être différents selon la réaction d'hydrocyanation mise en oeuvre.
En effet, la fabrication de dinitriles est généralement réalisée en deux étapes successives consistant dans une première étape à hydrocyaner avec HCN une dioléfine telle que le butadiène en mononitriles insaturés et dans une seconde étape à transformer ces mononitriles en dinitriles par réaction avec HCN. De plus, le procédé comprend généralement une étape d'isomérisation des mononitriles insaturés ramifiés obtenus en première étape pour les transformer en mononitriles linéaires qui seront transformés en dinitriles linéaires dans la seconde étape.

Ainsi, le rapport ligand/nickel utilisé en première étape est généralement plus élevé que celui utilisé dans la deuxième étape. De même, la concentration en nickel peut être différente dans les deux étapes.
Par ailleurs, dans la réaction d'hydrocyanation des nitriles insaturés ou seconde étape, un promoteur ou cocatalyseur est généralement utilisé. Comme promoteurs préférés, les acides de Lewis sont généralement choisis.

A titre d'exemple, On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont avantageusement choisis parmi les composés des éléments des groupes Ib, IIb, IIIa IIIb, IVa, IVb, Va, Vb, Vlb, Vllb et VIII de la Classification périodique des éléments. Ces composés sont le plus souvent des sels, notamment des halogénures, comme chlorures ou bromures, sulfates, sulfonates, halogenoalkylsulfonates, perhalogénoalkylsulfonates, notamment fluoroalkylsulfonates ou perfluoroalkylsulfonates, halogénoacétates, perhalogénoacétates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le chlorure d'indium, le trifluorométhylsulfonate d'indium, le trifluoroacétate d'indium, les chlorures ou bromures des éléments des terres rares comme le lanthane, le cérium, le praséodyme, le néodyme, le samarium, l'europium, le gadolinium; le terbium, le dysprosium, l'hafnium, l'erbium, le thullium, l'ytterbium et le lutétium, le chlorure de cobalt, le chlorure ferreux, le chlorure d'yttrium.

On peut également utiliser comme acide de Lewis des composés comme le triphénylborane, le tétraisopropylate de titane.

On peut bien entendu mettre en oeuvre des mélanges de plusieurs acides de Lewis.
Parmi les acides de Lewis, on préfère tout particulièrement le chlorure de zinc, le bromure de zinc, le chlorure stanneux, le bromure stanneux, le triphénylborane, le trifluorométhylsulfonate d'indium, le trifluoroacétate d'indium, et les mélanges chlorure de zinc/chlorure stanneux.
Le cocatalyseur acide de Lewis mis en oeuvre représente généralement de 0,005 à 50 moles par mole de nickel.

Dans chacune de ces étapes, le milieu réactionnel obtenu après la réaction d'hydrocyanation est avantageusement soumis à une séparation par distillation du réactif qui n'a pas réagi, à savoir le butadiène ou le nitrile insaturé, pour être recyclé.

Selon l'invention ces étapes de séparation sont réalisées en respectant une température de pied de distillation, suivant les conditions de rapport ligand/nickel et concentration en nickel indiquées précédemment pour éviter ou limiter la décomplexation du nickel et sa précipitation.

Selon des caractéristiques préférées de l'invention, le rapport ligand total/nickel exprimé en nombre d'atome de phosphore par rapport au nombre d'atome de nickel, est de préférence inférieur ou égal à 15, avantageusement compris entre 5 et 15, la concentration pondérale en nickel étant avantageusement comprise entre 0,1 % et 2% de préférence entre 0,1 % et 1,2 % (bornes incluses).

Par ligand total, on entend la totalité des molécules de ligands monodentates et bidentates. Dans le présent texte l'expression ligand/nickel se rapporte toujours au rapport de la totalité des molécules de ligands mono et bidentates par rapport au.nombre d'atome de nickel, sauf indication explicite différente.

Ainsi, dans le cas de la séparation du butadiène ou des composés résiduels d'une coupe C4, par distillation du milieu réactionnel issu de la première étape d'hydrocyanation, la température maximale du pied de distillation est inférieure ou égale à 180°C pour un rapport de mole de ligand bidentate, exprimé en atome de phosphore au nombre d'atome de nickel compris entre 1 et 4 (bornes incluses) et celui du ligand monodentate compris entre 4 et 12 (bornes incluses). En outre, selon une caractéristique préférée de l'invention, la concentration pondérale en nickel est supérieure ou égale à 0,1%, avantageusement supérieure à 0,2 %

De même, dans l'étape de séparation des nitriles insaturés à partir du milieu issu de la seconde étape d'hydrocyanation des nitriles insaturés, la température de pied de distillation est avantageusement inférieure ou égale à 140°C pour un rapport de mole de ligand bidentate, exprimé en atome de phosphore au nombre d'atome de nickel compris entre 1 et 4 (bornes incluses) et celui du ligand monodentate compris entre 4 et 7 (bornes incluses). En outre, selon une caractéristique préférée de l'invention, la concentration pondérale en nickel est supérieure ou égale à 0,2%.

Par ailleurs, dans un mode de réalisation, le procédé de l'invention peut comprendre une étape d'extraction du complexe organométallique et des ligands organophosphorés du milieu contenant les nitriles et en particulier les dinitriles par une extraction liquide/liquide au moyen d'un solvant d'extraction. Comme solvant d'extraction convenable, on peut citer à titre d'exemple, les hydrocarbures aliphatiques saturés linéaires ou cycliques comme l'hexane, l'heptane, l'octane, le cyclohexane, le cyclopentane, le cycloheptane et plus généralement les cycloparaffines ou analogues. Le cyclohexane est le solvant d'extraction préféré.

La solution de ligands et de complexe organométallique dans le solvant d'extraction est également soumise à une distillation pour séparer le solvant d'extraction et recycler les ligands et le complexe organométallique ainsi récupérés. Dans la présente invention, le solvant d'extraction est un réactif utilisé pour la mise en oeuvre du procédé.

Cette séparation est également effectuée en respectant les caractéristiques de l'invention indiquées précédemment telles que rapport ligand/nickel, concentration en nickel et température de pied de distillation.

Selon un mode de réalisation préféré de l'invention, cette séparation du cyclohexane est effectuée avec une température de pied de distillation inférieure ou égale à 180°C pour une concentration pondérale en nickel supérieure ou égale à 0,7%. En outre, selon une caractéristique préférée de l'invention, le rapport de mole de ligand bidentate, exprimé en atome de phosphore au nombre d'atome de nickel est compris entre 1 et 4 (bornes incluses) et celui du ligand monodentate est supérieur ou égal à 8.

Selon l'invention, dans les étapes de séparation par distillation à partir d'un milieu contenant le système catalytique, la température maximale du pied de distillation est déterminée en fonction du rapport ligand/nickel et de la concentration en nickel dans le milieu. Ainsi cette température est d'autant plus élevée que le rapport ligand/nickel et/ou la concentration en nickel sont élevés.

Dans un mode de réalisation préféré de l'invention, le procédé comprend une étape de séparation du système catalytique et des dinitriles réalisée dans une étape de décantation en deux phases dense et légère. Le milieu alimenté dans cette étape de décantation est soit le milieu obtenu après la seconde étape d'hydrocyanation soit le pied de distillation obtenu à l'étape de séparation des nitriles insaturés n'ayant pas réagi.

Ainsi, la phase inférieure ou dense comprend la majeure partie du nickel et du ligand bidentate et une partie du ligand monodentate , la phase supérieure ou légère étant constituée par les dinitriles et la partie restante du nickel et des ligands mono et bidentate.

Le milieu alimenté dans l'étape de décantation est avantageusement refroidi à une température comprise entre 25 et 75 °C, de préférence entre 30 et 55°C.

Selon une autre caractéristique du procédé de l'invention, la phase supérieure récupérée à l'étape de décantation contient un rapport molaire ligand/nickel avantageusement supérieur à 8.

Selon l'invention, la récupération totale du complexe métallique et des ligands est effectuée par une extraction liquide/liquide de ceux-ci au moyen d'un solvant d'extraction non miscible avec les dinitriles. Cette opération correspond à celle décrite précédemment, les solvants étant identiques.

Dans ce mode de réalisation, il est avantageux, préalablement à l'étape de distillation des nitriles insaturés n'ayant pas réagi, d'ajuster et de contrôler la concentration en nickel dans le milieu par addition d'une partie de-la phase inférieure ou dense récupérée à l'étape de décantation.

En effet, la concentration en nickel utilisé dans le milieu d'hydrocyanation quand il est associé à un ligand bidentate conformément au procédé de l'invention, peut être très faible comme, par exemple, de l'ordre de 100 à 2000 mg de nickel/kg de milieu réactionnel. Pour obtenir une concentration en nickel convenable pour favoriser la décantation, il peut être nécessaire d'ajouter une certaine quantité de système catalytique dans le milieu réactionnel sortant de l'étape d'hydrocyanation. Cet ajustement de la concentration en nickel peut être effectuée comme indiqué précédemment.

Le procédé de l'invention permet donc une récupération et séparation du catalyseur avec une perte d'élément métallique et donc de catalyseur minimisée.

La présente invention s'applique de manière préférentielle à l'hydrocyanation de butadiène et de nitriles insaturés linéaires ou ramifiés comprenant de 3 à 8 atomes de carbone et plus préférentiellement de 3-pentenenitrile et/ou 4-penténenitrile pour la production d'adiponitrile avec utilisation d'un catalyseur du type représenté par la formule suivante :

**Ni (L₁)ₓ (L₂)_{y}**

Dans laquelle L₁ représente un ligand monodentate et L₂ un ligand bidentate.
x et y représentent des nombres décimaux allant de 0 à 4, la somme x +2y étant égale à 3 ou 4. Le catalyseur peut être constitué d'un mélange de complexes répondant à la formule générale ci-dessus.

Le système catalytique ou le milieu réactionnel peut également comprendre une quantité de ligand organophosphorés mono et/ou bidentate sous forme libre, c'est-à-dire non lié au nickel Les systèmes catalytiques de l'invention peuvent être obtenus par formation dans une première étape d'un complexe organométallique entre le nickel et le ligand monodentate. Des procédés de formation de tels complexes sont par exemple, décrits dans les brevets US 3903120 et US 4,416,825.
Dans une seconde étape, le ligand bidentate est ajouté dans le milieu contenant le dit complexe organométallique.

Les première et seconde étapes d'hydrocyanation sont avantageusement réalisées en série. Dans ce cas, il est avantageux qu'au moins une partie du catalyseur récupéré, notamment celui récupéré à l'extraction liquide/liquide, et plus particulièrement le catalyseur récupéré dans la phase supérieure de l'étape de décantation, soit recyclée et utilisée comme catalyseur dans la première étape d'hydrocyanation du butadiène, et/ou à l'étape d'isomérisation des nitriles insaturés ramifiés en nitriles insaturés linéaires. L'utilisation d'un système catalytique identique ou similaire pour l'hydrocyanation du butadiène et celle des pentènenitriles est préférée.

Toutefois, il est également possible d'utiliser le système catalytique décrit ci-dessus uniquement dans l'étape d'hydrocyanation des nitriles insaturés, le système catalytique utilisé dans la première étape d'hydrocyanation du butadiène et l'étape d'isomérisation étant différent par la nature des composés.

D'autres détails, avantages de l'invention apparaîtront plus clairement au vu des exemples donnés ci-dessous uniquement à titre d'illustration.

### Abréviations utilisées dans les exemples:

PN : pentènenitriles
DN : dinitriles (mélange de dinitriles AdN, MGN, ESN contenant majoritairement de l'AdN)
AdN: adiponitrile
MGN : méthylglutaronitrile
ESN : éthylsuccinonitrile
Ligands:

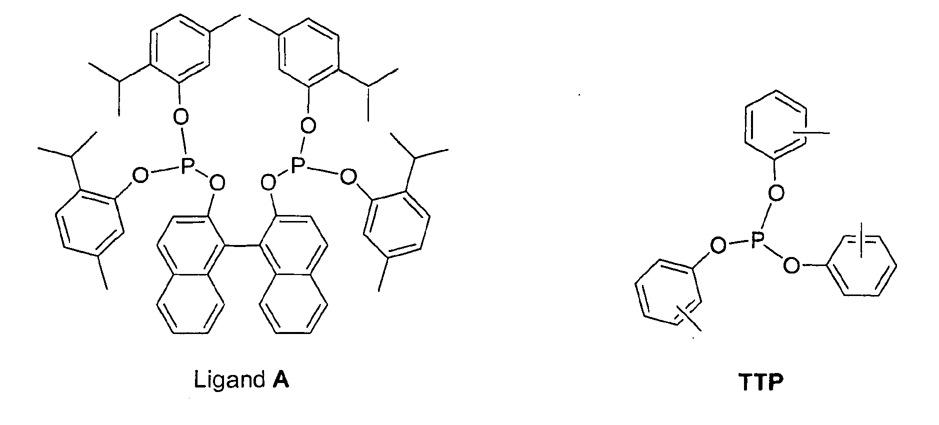

### Exemple 1 : Distillation des pentènenitriles (PN)

Sous atmosphère inerte, on prépare un mélange de composition suivante (les pourcentages son exprimés en poids, les rapports sont des rapports molaires):
Ni = 1,2%
TTP/Ni = 5
Ligand A/Ni = 1
P/Ni = 7
DN = 38.5%
PN = 5%

Ce mélange est introduit dans un tube qui est scellé et chauffé pendant 2h à une certaine température (voir tableau), puis on détermine la perte en nickel complexé par analyse chromatographique :

| Température | Perte Ni |
|---|---|
| 130°C | 0% |
| 150°C | 25% |

### Exemple 2 : Etape de distillation de la solution obtenue après extraction liquide/liquide

Sous atmosphère inerte, on prépare un mélange de composition suivante (les pourcentages son exprimés en poids, les rapports sont des rapports molaires) :
Ni =1,0%
TTP/Ni = 12
Ligand A/Ni = 1
P/Ni = 13
PN = 11 %

Ce mélange est introduit dans un tube qui est scellé et chauffé pendant 2h à une certaine température (voir tableau) et on détermine la perte en nickel complexé par analyse chromatographique :

| Température | Perte Ni |
|---|---|
| 170°C | 0% |
| 190°C | 45% |

## Revendications

1. Procédé d'hydrocyanation de composés insaturés comprenant au moins une étape d'hydrocyanation en présence d'un système catalytique comprenant un complexe organométallique formé par un ou plusieurs ligands organophosphorés de type organophosphite monodentate, et un ou plusieurs ligands organophosphorés bidentates, et un élément métallique choisi dans le groupe comprenant le nickel, le cobalt, le fer, le ruthénium, le rhodium, le palladium, l'osmium, l'iridium, le platine, le cuivre, l'argent, l'or, le zinc, le cadmium, le cérium et comprenant éventuellement un promoteur de type acide de Lewis, et au moins une étape de séparation par distillation d'un réactif utilisé dans le procédé ou d'un composé formé au cours de la réaction d'hydrocyanation, à partir d'un milieu contenant ledit système catalytique, **caractérisé en ce que** le milieu soumis à l'étape de séparation par distillation comprend un rapport du nombre de moles de ligands organophosphorés exprimé en atome de phosphore par rapport au nombre d'atome d'élément métallique inférieur ou égal à 15 et/ou une concentration pondérale en élément métallique formant le complexe organométallique inférieure ou égale à 1,3%, et **en ce que** la température de pied de distillation de l'étape de distillation est inférieure ou égale à 180°C.

2. Procédé selon la revendication 1, **caractérisé en ce que** le ligand monodentate est un composé choisi dans le groupe comprenant le triphénylphosphite, le tritolylphosphite, le tricyménylphosphite.

3. Procédé selon la revendication 1 ou 2, **caractérisé en ce que** le ligand organophosphoré bidentate est choisi dans le groupe comprenant les organophosphites organophosphonites, organophosphinites, organophosphines.

4. Procédé selon la revendication 3, **caractérisé en ce que** le ligand organophosphoré bidentate est choisi dans le groupe comprenant les composés de structures suivantes dans lesquelles Ph signifie phényle :

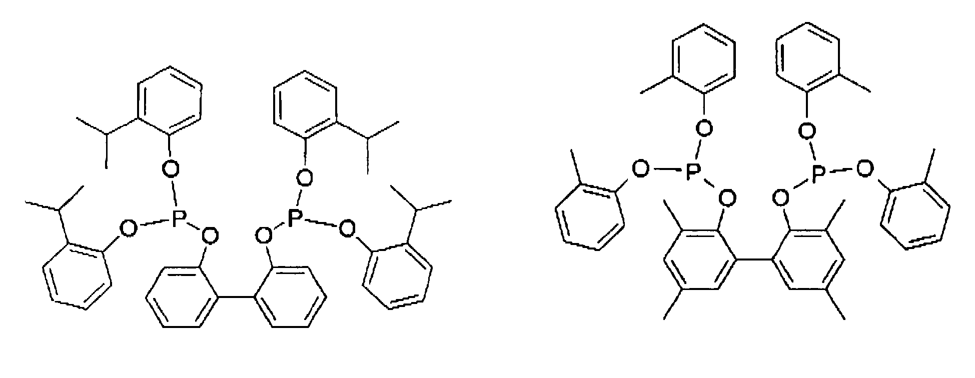

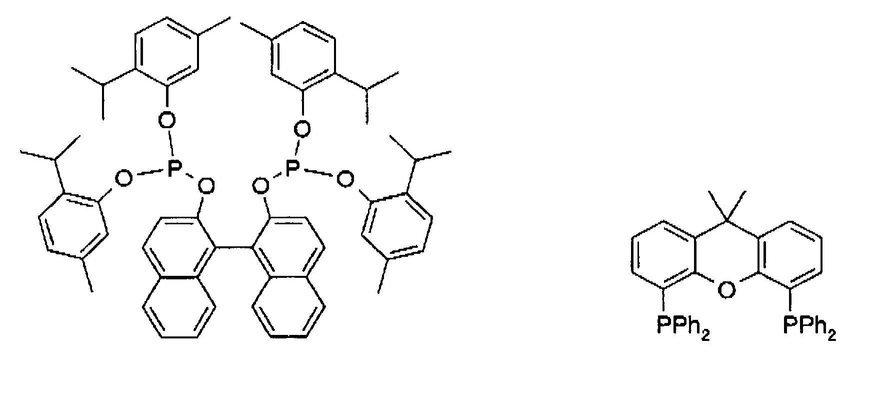

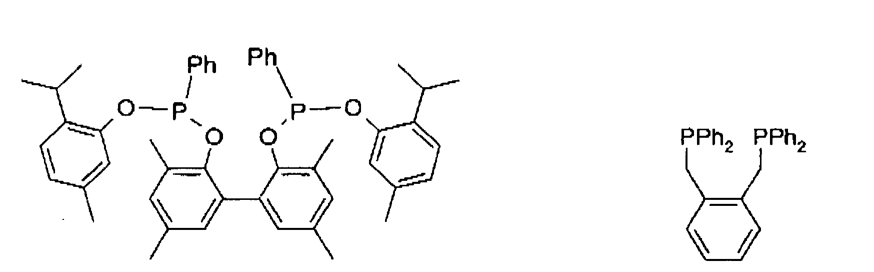

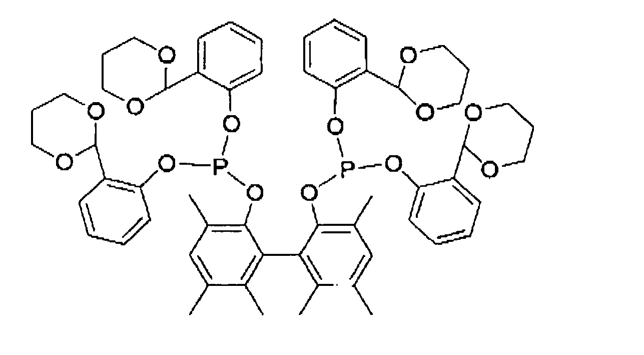

5. procédé selon l'une des revendications précédentes, **caractérisé en ce que** le promoteur est choisi dans le groupe comprenant les composés des éléments des groupes Ib, IIb, IIIa, IIIb, IVa, IVb, Va, Vb, VIb, VIIb et VIII de la Classification périodique des éléments, lesdits composés étant choisis dans le groupe comprenant les halogénures, sulfates, sulfonates, halogenoalkylsulfonates, perhalogénoalkylsulfonates, halogénoacétates, perhalogénoacétates, carboxylates, phosphates, les aryles boranes.

6. Procédé selon la revendication 5, **caractérisé en ce que** l'acide de Lewis est choisi dans le groupe comprenant le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le chlorure d'indium, le trifluorométhylsulfonate d'indium, le trifluoroacétate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thullium, l'ytterbium et le lutétium, le chlorure de cobalt, le chlorure ferreux, le chlorure d'yttrium, le triphényl borane, le tétraisopropylate de titane et leurs mélanges

7. Procédé selon l'une des revendications précédentes **caractérisé en ce que** le composé insaturé est le butadiène et/ou un composé nitrile insaturé.

8. Procédé selon l'une des revendications précédentes **caractérisé en ce que** le nitrile insaturé est un composé aliphatique linéaire ou ramifié comprenant de 3 à 8 atomes de carbone

9. Procédé selon la revendication 7, **caractérisé en ce que** le nitrile insaturé est un pentènenitrile.

10. Procédé selon l'une des revendications précédentes, **caractérisé en ce que** l'étape de séparation par distillation est une étape de distillation du milieu issu d'une étape d'hydrocyanation pour séparer le composé insaturé n'ayant pas réagi.

11. Procédé selon l'une des revendications précédentes **caractérisé en ce que** la récupération du système catalytique des dinitriles formés comprend une étape de décantation du milieu réactionnel issu de l'hydrocyanation d'un nitrile insaturé, après éventuellement séparation par distillation du nitrile insaturé n'ayant pas réagi.

12. Procédé selon la revendication 10 ou 11, **caractérisé en ce que** le milieu réactionnel issu de l'hydrocyanation d'un nitrile insaturé après éventuellement séparation dudit mononitrile n'ayant pas réagi ou la phase supérieure ou légère récupérée à l'étape de décantation est soumise à une extraction liquide/liquide pour extraire les ligands organophosphorés et le complexe organométallique présents dans lesdits milieux.

13. Procédé selon la revendication 12, **caractérisé en ce que** le solvant d'extraction est choisi dans le groupe comprenant l'hexane, l'heptane, octane, heptane, le cyclohexane, le cyclopentane, le cycloheptane.

14. Procédé selon l'une des revendications 10 à 12, **caractérisé en ce que** l'étape de séparation est une étape de distillation du solvant d'extraction de la solution obtenue après l'extraction liquide/liquide.

15. Procédé selon l'une des revendications 10 à 14, **caractérisé en ce qu'**une partie de ladite phase inférieure est recyclée dans le milieu réactionnel issu de l'hydrocyanation, avant l'étape de distillation des nitriles insaturés.

16. Procédé selon l'une des revendications précédentes, **caractérisé en ce que** l'élément métallique est le nickel

## Claims

1. Process for the hydrocyanation of unsaturated compounds comprising at least one stage of hydrocyanation in the presence of a catalytic system comprising an organometallic complex formed by one or more organophosphorus ligands of monodentate organophosphite type and one or more bidentate organophosphorus ligands and a metal element chosen from the group consisting of nickel, cobalt, iron, ruthenium, rhodium, palladium, osmium, iridium, platinum, copper, silver, gold, zinc, cadmium and cerium and optionally comprising a promoter of Lewis acid type and at least one stage of separation by distillation of a reactant used in the process or of a compound formed during the hydrocyanation reaction from a medium comprising the said catalytic system, **characterized in that** the medium subjected to the stage of separation by distillation comprises a ratio of the number of moles of organophosphorus ligands, expressed as phosphorus atoms, with respect to the number of metal element atoms of less than or equal to 15 and/or a concentration by weight of metal element forming the organometallic complex of less than or equal to 1.3% and **in that** the distillation bottom temperature of the distillation stage is less than or equal to 180°C.

2. Process according to Claim 1, **characterized in that** the monodentate ligand is a compound chosen from the group consisting of triphenyl phosphite, tritolyl phosphite and tricymenyl phosphite.

3. Process according to Claim 1 or 2, **characterized in that** the bidentate organophosphorus ligand is chosen from the group consisting of organophosphites, organophosphonites, organophosphinites and organophosphines.

4. Process according to Claim 3, **characterized in that** the bidentate organophosphorus ligand is chosen from the group consisting of the compounds with the following structures, in which Ph means phenyl:

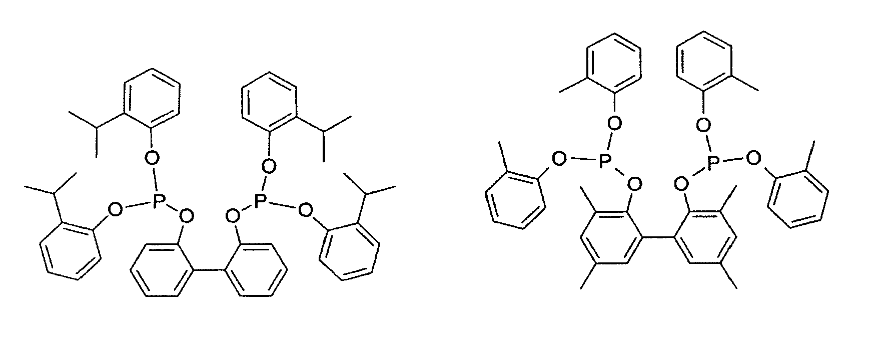

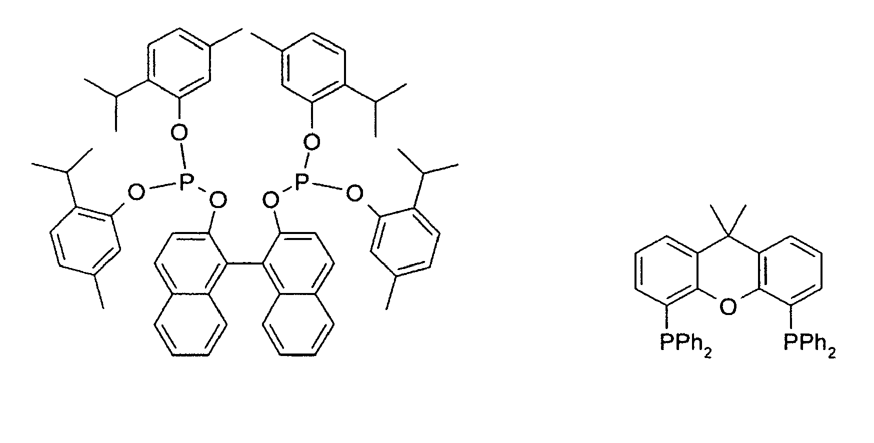

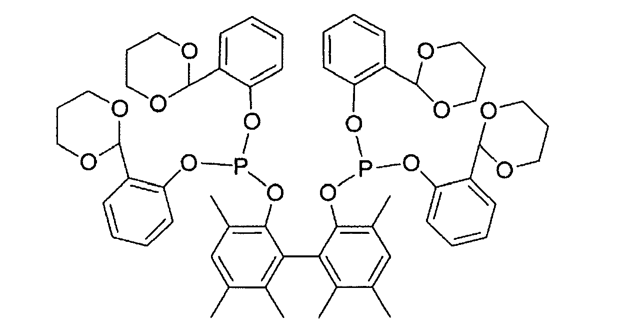

5. Process according to one of the preceding claims, **characterized in that** the promoter is chosen from the group consisting of compounds of the elements from Groups Ib, IIb, IIIa, IIIb, IVa, IVb, Va, Vb, VIb, VIIb and VIII of the Periodic Table of the Elements, the said compounds being chosen from the group consisting of halides, sulphates, sulphonates, haloalkylsulphonates, perhaloalkylsulphonates, haloacetates, perhaloacetates, carboxylates and phosphates, and arylboranes.

6. Process according to Claim 5, **characterized in that** the Lewis acid is chosen from the group consisting of zinc chloride, zinc bromide, zinc iodide, manganese chloride, manganese bromide, cadmium chloride, cadmium bromide, stannous chloride, stannous bromide, stannous sulphate, stannous tartrate, indium chloride, indium trifluoromethylsulphonate, indium trifluoroacetate, chlorides or bromides of rare earth elements, such as lanthanum, cerium, praseodymium, neodymium, samarium, europium, gadolinium, terbium, dysprosium, hafnium, erbium, thulium, ytterbium and lutetium, cobalt chloride, ferrous chloride, yttrium chloride, triphenylborane, titanium tetraisopropoxide and their mixtures.

7. Process according to one of the preceding claims, **characterized in that** the unsaturated compound is butadiene and/or an unsaturated nitrile compound.

8. Process according to one of the preceding claims, **characterized in that** the unsaturated nitrile is a linear or branched aliphatic compound comprising from 3 to 8 carbon atoms.

9. Process according to Claim 7, **characterized in that** the unsaturated nitrile is a pentenenitrile.

10. Process according to one of the preceding claims, **characterized in that** the stage of separation by distillation is a stage of distillation of the medium resulting from a hydrocyanation stage in order to separate the unreacted unsaturated compound.

11. Process according to one of the preceding claims, **characterized in that** the recovery of the catalytic system from the dinitriles formed comprises a stage of settling the reaction medium resulting from the hydrocyanation of an unsaturated nitrile, optionally after separation by distillation of the unreacted unsaturated nitrile.

12. Process according to Claim 10 or 11, **characterized in that** the reaction medium resulting from the hydrocyanation of an unsaturated nitrile, optionally after separation of the said unreacted mononitrile, or the upper or light phase recovered in the settling stage is subjected to liquid/liquid extraction in order to extract the organophosphorus ligands and the organometallic complex present in the said media.

13. Process according to Claim 12, **characterized in that** the extraction solvent is chosen from the group consisting of hexane, heptane, octane, cyclohexane, cyclopentane and cycloheptane.

14. Process according to one of Claims 10 to 12, **characterized in that** the separation stage is a stage of distillation of the extraction solvent from the solution obtained after liquid/liquid extraction.

15. Process according to one of Claims 10 to 14, **characterized in that** a portion of the said lower phase is recycled in the reaction medium resulting from the hydrocyanation, before the stage of distillation of the unsaturated nitriles.

16. Process according to one of the preceding claims, **characterized in that** the metal element is nickel.

## Patentansprüche

1. Verfahren zur Hydrocyanierung von ungesättigten Verbindungen, umfassend mindestens eine Stufe zur Hydrocyanierung in Anwesenheit eines katalytischen Systems, das einen organometallischen Komplex umfasst, gebildet aus einem oder mehreren Organophosphor-Liganden vom Typ einzähniger Organophosphite, und einem oder mehreren zweizähnigen Organophosphor-Liganden, und einem metallischen Element, gewählt aus der Gruppe, die Nickel, Cobalt, Eisen, Ruthenium, Rhodium, Palladium, Osmium, Iridium, Platin, Kupfer, Silber, Gold, Zink, Cadmium, Cerium und gegebenenfalls einen Promotor vom Typ Lewis-Säure umfasst, und mindestens eine Stufe zur Abtrennung durch Destillation eines im Verfahren verwendeten Reaktanden oder einer im Verlauf der Reaktion der Hydrocyanierung gebildeten Verbindung ausgehend von einem Medium, welches das genannte katalytische System umfasst, **dadurch gekennzeichnet, dass** das Medium, das der Stufe zur Abtrennung durch Destillation unterzogen wird, ein Verhältnis der Anzahl von Molen der Organophosphor-Liganden, ausgedrückt in Phosphoratomen, bezogen auf die Anzahl der Atome des metallischen Elementes von unter oder gleich 15 und/oder eine gewichtsmäßige Konzentration an metallischem Element, das den organometallischen Komplex bildet, von unter oder gleich 1,3 % umfasst, und **dadurch**, **dass** die Temperatur des Destillationssumpfes der Stufe der Destillation unter oder gleich 180 °C beträgt.

2. Verfahren nach Anspruch 1, **dadurch gekennzeichnet, dass** der einzähnige Ligand eine Verbindung ist, gewählt aus der Gruppe, die Triphenylphosphit, Tritolylphosphit, Tricymenylphosphit umfasst.

3. Verfahren nach Anspruch 1 oder 2, **dadurch gekennzeichnet, dass** der zweizähnige Organophosphor-Ligand aus der Gruppe gewählt wird, die Organophosphite, Organophosphonite, Organophosphinite, Organophosphine umfasst.

4. Verfahren nach Anspruch 3, **dadurch gekennzeichnet, dass** der zweizähnige Organophosphor-Ligand aus der Gruppe gewählt wird, welche die Verbindungen der folgenden Strukturen umfasst, in denen Ph Phenyl bezeichnet:

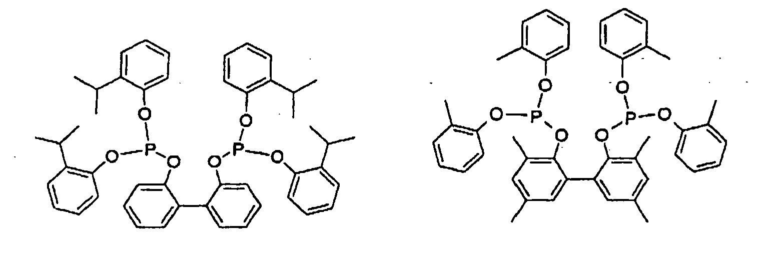

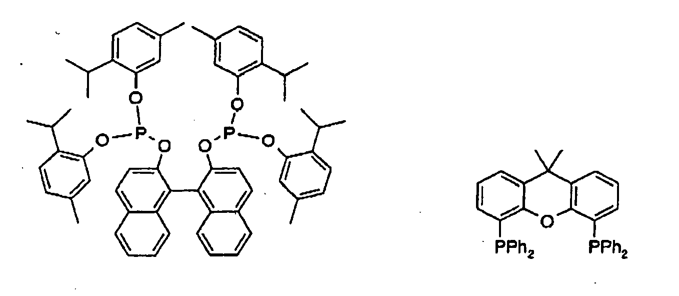

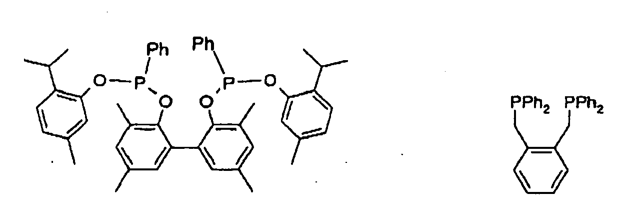

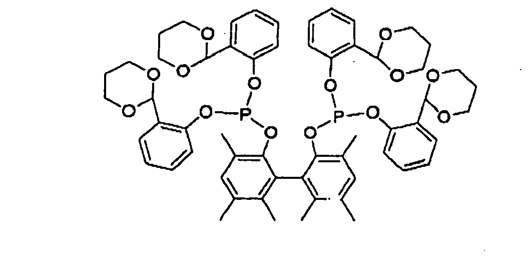

5. Verfahren nach einem der vorstehenden Ansprüche, **dadurch gekennzeichnet, dass** der Promotor aus der Gruppe gewählt wird, welche die Verbindungen der Elemente der Gruppen Ib, IIb, II-Ia, IIIb, IVa, IVb, Va, Vb, VIb, VIIb und VIII des Periodensystems der Elemente umfasst, wobei die genannten Verbindungen aus der Gruppe gewählt werden, welche die Halogenide, Sulfate, Sulfonate, Halogenalkylsulfonate, Perhalogenalkylsulfonate, Halogenacetate, Perhalogenacetate, Carboxylate, Phosphate, die Arylborane umfasst.

6. Verfahren nach Anspruch 5, **dadurch gekennzeichnet, dass** die Lewis-Säure aus der Gruppe gewählt wird, die Zinkchlorid, Zinkbromid, Zinkiodid, Manganchlorid, Manganbromid, Cadmiumchlorid, Cadmiumbromid, Zinn(II)-chlorid, Zinn(II)-bromid, Zinn(II)-sulfat, Zinn(II)-tartrat, Indiumchlorid, Indium-trifluormethylsulfonat, Indium-trifluoracetat, die Chloride oder Bromide der Elemente der Seltenen Erden wie Lanthan, Cerium, Praseodym,
Neodym, Samarium, Europium, Gadolinium, Terbium, Dysprosium, Hafnium, Erbium, Thulium, Ytterbium und Lutetium, Cobaltchlorid, Eisen(II)-chlorid, Yttriumchlorid, Triphenylboran, Titantetraisopropylat und ihre Mischungen umfasst.

7. Verfahren nach einem der vorstehenden Ansprüche, **dadurch gekennzeichnet, dass** die ungesättigte Verbindung Butadien und/ oder eine ungesättigte Nitrilverbindung ist.

8. Verfahren nach einem der vorstehenden Ansprüche, **dadurch gekennzeichnet, dass** das ungesättigte Nitril eine lineare oder verzweigte aliphatische Verbindung mit 3 bis 8 Kohlenstoffatomen ist.

9. Verfahren nach Anspruch 7, **dadurch gekennzeichnet, dass** das ungesättigte Nitril ein Penten-nitril ist.

10. Verfahren nach einem der vorstehenden Ansprüche, **dadurch gekennzeichnet, dass** die Stufe der Trennung durch Destillation eine Stufe der Destillation des Mediums ist, das von einer Stufe der Hydrocyanierung stammt, um die ungesättigte Verbindung abzutrennen, die nicht reagiert hat.

11. Verfahren nach einem der vorstehenden Ansprüche, **dadurch gekennzeichnet, dass** die Wiedergewinnung des katalytischen Systems von den gebildeten Dinitrilen eine Stufe der Dekantierung des Reaktionsmediums umfasst, das von der Hydrocyanierung eines ungesättigten Nitrils stammt, gegebenenfalls nach der Abtrennung des nicht umgesetzten ungesättigten Nitrils durch Destillation.

12. Verfahren nach Anspruch 10 oder 11, **dadurch gekennzeichnet, dass** das Reaktionsmedium, das von der Hydrocyanierung eines ungesättigten Nitrils stammt, gegebenenfalls nach der Abtrennung des genannten, nicht umgesetzten Mononitrils oder der bei der Stufe der Dekantierung gewonnenen oberen oder leichteren Phase, einer Flüssig-Flüssig-Extraktion unterzogen wird, um die Organophosphor-Liganden und die organometallischen Komplexe zu extrahieren, die in den genannten Medien vorhanden sind.

13. Verfahren nach Anspruch 12, **dadurch gekennzeichnet, dass** das Lösungsmittel der Extraktion aus der Gruppe gewählt wird, die Hexan, Heptan, Octan, Heptan, Cyclohexan, Cyclopentan, Cycloheptan umfasst.

14. Verfahren nach einem der Ansprüche 10 bis 12, **dadurch gekennzeichnet, dass** die Stufe der Trennung eine Stufe.der Destillation des Lösungsmittels der Extraktion von der Lösung ist, die nach der Flüssig-Flüssig-Extraktion erhalten wird.

15. Verfahren nach einem der Ansprüche 10 bis 14, **dadurch gekennzeichnet, dass** ein Teil der genannten unteren Phase in dem Reaktionsmedium, das von der Hydrocyanierung stammt, vor der Stufe der Destillation der ungesättigten Nitrile zurückgewonnen wird.

16. Verfahren nach einem der vorstehenden Ansprüche, **dadurch gekennzeichnet, dass** das metallische Element Nickel ist.
